# EUROPEAN PATENT SPECIFICATION

(11) **EP 1 686 970 B1**
(45) Date of publication and mention of the grant of the patent: **10.04.2019**
(21) Application number: 04798381.2
(22) Date of filing: 03.11.2004
(51) Int. Cl.: A61K 39/00, A61P 1/00, C07K 16/24

(54) **Use of an inhibitor of CSF-1 activity for the treatment of Crohn's disease or ulcerative colitis**
Verwendung eines Inhibitors der CSF-1 Wirkung zur Behandlung der Crohn-Krankheit oder der Colitis ulcerosa
Utilisation d'un inhibiteur de l'activité du CSF-1 dans le traitement de la maladie de Crohn ou de la colite ulcéreuse

(30) Priority: 05.11.2003 GB 0325836
(43) Date of publication of application: 09.08.2006
(73) Proprietor: UCB Biopharma SPRL, 1070 Brussels (BE)
(72) Inventor: LAWSON, Alastair, David, G., Celltech R & D Ltd., Slough, Berkshire SL1 3WE (GB); BOURNE, Timothy, Celltech R & D Limited, Slough, Berkshire SL1 3WE (GB)
(74) Representative: Thompson, John
(86) International application number: PCT/GB2004/004652
(87) International publication number: WO 2005/046657

(56) References cited:
- WO-A-00/67777
- WO-A-01/30381
- WO-A-99/43839
- WO-A-02/087496
- WO-A-03/020698
- WO-A-2005/010009
- WO-A-2005/012226
- WO-A-2005/030124
- US-A- 5 932 595
- US-A1- 2002 010 126
- US-A1- 2002 141 994
- MAROTTA F ET AL: "Disrupted mucosal barrier in quiescent ulcerative colitis: Effect of metronidazole and of a symbiotic preparation in a pilot cross-over study" CHINESE JOURNAL OF DIGESTIVE DISEASES 2003 AUSTRALIA, vol. 4, no. 4, 2003, pages 180-185, XP009051465 ISSN: 1443-9611
- KLEBL F H ET AL: "Expression of macrophage-colony stimulating factor in normal and inflammatory bowel disease intestine" JOURNAL OF PATHOLOGY 2001 UNITED KINGDOM, vol. 195, no. 5, 2001, pages 609-615, XP002339478 ISSN: 0022-3417
- MAKIYAMA K ET AL: "Serum concentration of macrophage colony stimulating factor (M-CSF) in patients with inflammatory bowel disease" GASTROENTEROLOGIA JAPONICA 1993 JAPAN, vol. 28, no. 5, 1993, page 740, XP009051960 ISSN: 0435-1339

## Description

The present invention relates generally to the use of inhibitors of CSF-1 activity for the manufacture of a medicament for the treatment of inflammatory bowel disease.

The colony stimulating factor 1 (CSF-1), also known as macrophage colony stimulating factor (M-CSF) is a cytokine produced by a variety of cells, including macrophages, endothelial cells and fibroblasts. CSF-1 is composed of two "monomer" polypeptides, which form a biologically active dimeric CSF-1 protein. CSF-1 exists in at least three mature forms due to alternative mRNA splicing (see, Cerretti et al. Molecular Immunology, 25:761 (1988)). The three forms of CSF-1 are translated from different mRNA precursors, which encode polypeptide monomers of 256 to 554 amino acids, having a 32 amino acid signal sequence at the amino terminal and a putative transmembrane region of approximately 23 amino acids near the carboxyl terminal. The precursor peptides are subsequently processed by amino terminal and carboxyl terminal proteolytic cleavages to release mature CSF-1. Residues 1-149 of all three mature forms of CSF-1 are identical and are believed to contain sequences essential for biological activity of CSF-1. In vivo CSF-1 monomers are dimerized via disulfide-linkage and are glycosylated. CSF-1 belongs to a group of biological agonists that promote the production of blood cells. Specifically, it acts as a growth and differentiation factor for bone marrow progenitor cells of the mononuclear phagocyte lineage. Further, CSF-1 stimulates the proliferation and function of mature macrophages via specific receptors on responding cells. The CSF-1 receptor is also referred to as the c-fms gene product or CD115. For review see Roth and Stanley, 1992, Current Topics in Microbiology and Immunology, 181, 141-167.

The term 'inflammatory bowel disease' (IBD) refers to serious, chronic disorders of the intestinal tract characterised by chronic inflammation at various sites in the gastrointestinal tract, and specifically includes ulcerative colitis (UC) and Crohn's disease (CD). The cause of IBD is unknown but is most commonly thought to arise from inappropriate and ongoing activation of the mucosal immune system driven by the presence of normal luminal flora. This aberrant response is most likely facilitated by defects in both the barrier function of the intestinal epithelium and the mucosal immune system and both genetic and environmental factors are thought to contribute to susceptibility to IBD (Podolsky, 2002, N.Engl.J.Med, 347,6, 417-429). Treatment of IBD typically begins with steroids and 5-aminosalicylic acid drugs, although there are problems associated with their long-term use. In addition approximately 30% of IBD patients do not respond well to either therapy and require other treatments such as immunosuppressive and immunoregulatory agents and surgery (Sandborn, 1996, Am.J.Gastroenterol, 91, 423-432). The sustained activation of mucosal immune responses has led to extensive characterisation of immune cell populations and inflammatory mediators in patients with inflammatory bowel disease and in murine models. An increase in the number of intestinal macrophages has been noted in active CD and UC and monocytic cells appear to be involved in all stages of IBD (Fiocchi, 1998, Gastroenterology, 115, 182-205). Th1 cytokines are thought to activate macrophages, which in turn, produce interleukin-12, interleukin-18 and macrophage migration inhibitor factor and thus further stimulate Th1 in a self-sustaining cycle. In addition, the activated macrophages produce a potent mix of inflammatory cytokines, including TNF, interleukin-1 and interleukin-6 (Podolsky, 2002, N.Engl.J.Med, 347, 417-429). The mucosa of patients with established CD is dominated by CD4+ lymphocytes with a type I helper-T-cell (Th1) phenotype characterised by increased expression of interferon γ, interleukin (IL-)-2 followed by a subsequent increase in production of the proinflammatory cytokines TNF and IL-1β, and then NF-κB, as well as a compensatory increase in the Th2 mediated anti-inflammatory cytokine IL-10 and transforming growth factor β. In UC, the mucosa in patients may be dominated by CD4+ lymphocytes with an atypical type 2 helper-T-cell (Th2) phenotype, and a relatively decreased Th1 response. The Th2 response is characterised by an increased expression of IL-4, IL-5, IL-6, IL-10 and IL-13.

The many different cytokines and T-lymphocyte cell types implicated in IBD all represent potential targets for the treatment of IBD and many different treatments are in development whose targets include TNF, α4 integrin, IL-2, IL-12, interferon γ, ICAM-1 and CD-40 ligand (Sandborn, 2002, Gastroenterology, 122, 1592-1608).

Whether CSF-1 plays any role in the pathogenesis of IBD is not known. CSF-1 is known to have a role in macrophage production in the intestine as mice that lack CSF-1 lose the resident intestinal macrophage population (Cecchini et al., 1994, Development, 120, 1357-1372). Increased CSF-1 serum levels have been observed in patients with active IBD, in comparison to patients in remission or healthy controls (Makiyama et al., Gastroenterol. Jpn. 1993, 28, 740). In one study of inflamed IBD intestine a significant increase in the number of cells expressing both CSF-1 mRNA and protein in the lamina propria was observed; however there were no differences in overall CSF-1 mRNA levels in mucosal colonic biopsies and no correlation between mRNA levels and degree of mucosal inflammation in the biopsies was observed. None of the changes in frequency or cellular distribution of CSF-1 expression observed in the study were specific to IBD as there were similar findings in intestinal tuberculosis and ischaemic colitis (Klebl, 2001, Journal of Pathology, 195, 609-615). WO9943839 describes inhibitors of abnormal RPTK, WO0067777 describes a method of down regulating the functional level of endogenously produce molecules and US2002/141994 describes method of treating inflammation.

CSF-1 has been used in the treatment of immune suppression, in the treatment or prevention of bacterial, viral or fungal infections, the stimulation of white blood cells and in wound healing while inhibitors of CSF-1 have been used to treat tumor diseases (EP1223980). Inhibitors of CSF-1 activity are well known in the art, for example neutralising antibodies against CSF-1 and CSF-1R have been described (Weir et al., 1996, J Bone Miner. Res.11, 1474-1481; Haran-Ghera et al., 1997, Blood, 89, 2537-2545) and antisense antagonists of CSF-1 have also been described (EP1223980).

Surprisingly we have been able to demonstrate that inhibitors of CSF-1 activity are active in an animal model of IBD. Specifically we have been able to demonstrate that an anti-CSF-1 antibody that inhibits CSF-1 activity is active in an animal model of IBD. Accordingly, the present invention provides the use of an inhibitor of CSF-1 activity for the manufacture of a medicament for the treatment and/or prophylaxis of Crohn's disease or ulcerative colitis.

The term "inflammatory bowel disease" includes diseases that are characterised by chronic inflammation at various sites in the gastrointestinal tract. Illustrative inflammatory bowel diseases include but are not limited to regional enteritits (or Crohn's disease), idiopathic ulcerative colitis, idiopathic proctocolitis and infectious colitis.

The term 'CSF-1 activity' as used herein refers to the spectrum of activity understood in the art for CSF-1, in particular the activity of human CSF-1 i.e. when applied to the standard in vitro colony stimulating assay of Metcalf, J.Cell.Physiol (1970) 76-89.

An inhibitor of CSF-1 activity according to the present invention is an agent that interferes with the activity of CSF-1 in particular the activity of CSF-1 in Crohn's disease or ulcerative colitis. Particularly preferred are agents which interfere with the activity of CSF-1 in Crohn's disease or ulcerative colitis in humans. Inhibitors according to the present invention may partially or completely inhibit CSF-1 activity. Inhibitors of use in the present invention include without limitation, inhibitors that are capable of interacting with (*e.g*. binding to, or recognising) CSF-1 or the CSF-1 receptor (CSF-1 R) or a nucleic acid molecule encoding CSF-1 or CSF-1R, or are capable of inhibiting the expression of CSF-1 or CSF-1 R or are capable of inhibiting the interaction between CSF-1 and CSF-1R. Such inhibitors may be antibodies or functional active fragment thereof.

Also disclosed are inhibitors which include, but are not limited to, a synthetic functional fragment of the CSF-1 receptor that binds to CSF-1 and interferes with binding to the native CSF-1 receptor, an antibody that binds to CSF-1 or to the CSF-1 receptor and interferes with CSF-1 receptor-ligand interaction, an antisense nucleic acid molecule that specifically hybridizes to mRNA encoding CSF-1 or the CSF receptor or a small molecule or other drug which inhibits the activity of CSF-1 or its receptor.

Inhibitors of CSF-1 activity are well known in the art as are methods of identifying and producing such inhibitors. Neutralising anti-CSF-1 antibodies have been described, for example by Weir et al., 1996, J Bone Miner. Res.11, 1474-1481 and Haran-Ghera et al., 1997, Blood, 89, 2537-2545. The latter also describes anti-CSF-1R antibodies and antisense antagonists of CSF-1 have also been described (EP1223980). Also disclosed are agents that may be suitable inhibitors and can be selected from a wide variety of candidate agents. Examples of candidate agents include but are not limited to, nucleic acids (*e.g.* DNA and RNA), carbohydrates, lipids, proteins, polypeptides, peptides, peptidomimetics, small molecules and other drugs. Agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is suited to peptide libraries, while the other four approaches are applicable to peptide, non-peptide oligomer or small molecule libraries of compounds (Lam, 1997, Anticancer Drug Des. 12:145; U.S. 5,738,996; and U.S. 5,807,683).

Examples of suitable methods based on the present description for the synthesis of molecular libraries can be found in the art, for example in: DeWitt et al., 1993, Proc. Natl. Acad. Sci. USA 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994, J. Med. Chem. 37:1233.

Libraries of compounds may be presented, for example, in solution (*e.g*. Houghten, 1992, Bio/Techniques 13:412-421), or on beads (Lam, 1991, Nature 354:82-84), chips (Fodor, 1993, Nature 364:555-556), bacteria (US 5,223,409), spores (US 5,571,698; 5,403,484; and 5,223,409), plasmids (Cull et al., 1992, Proc. Natl. Acad. Sci. USA 89:1865-1869) or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J. Mol. Biol. 222:301-310).

Also disclosed is the inhibitor that may be a nucleic acid. In particular CSF-1 or CSF-1R nucleic acid molecules may be used as anti-sense molecules, to alter the expression of their respective polypeptides by binding to complementary nucleic acids. CSF-1 or CSF-1 R nucleic acids may be obtained using standard cloning techniques from genomic DNA or cDNA or can be synthesised using well known and commercially available techniques. The CSF-1 or CSF-1R nucleic acids may contain one or more nucleotide substitutions, additions or deletions into the nucleotide sequence of a CSF-1 or CSF-1R nucleic acid. Standard techniques known to those of skill in the art can be used to introduce mutations, including, site-directed mutagenesis and PCR-mediated mutagenesis. An antisense nucleic acid includes a CSF-1 or CSF-1R nucleic acid capable of hybridising by virtue of some sequence complementarity to a portion of an RNA (preferably mRNA) encoding the respective polypeptide. The antisense nucleic acid can be complementary to a coding and/or noncoding region of an mRNA encoding such a polypeptide. Most preferably, the antisense nucleic acids result in inhibition of the expression of the CSF-1 or CSF-1R polypeptide. Also disclosed is a method for the treatment and/or prophylaxis of IBD comprising administering a therapeutically effective amount of an inhibitor of CSF-1 activity wherein the inhibitor comprises at least eight nucleotides that are antisense to a gene or cDNA encoding a CSF-1 or CSF-1R polypeptide. Also disclosed is the use of nucleic acids comprising at least eight nucleotides that are antisense to a gene or cDNA encoding a CSF-1 or CSF-1R polypeptide for the manufacture of a medicament for use in the treatment and/or prophylaxis of IBD. Examples of sequences are provided in EP1223980.

According to the invention the inhibitor for use in the treatment and/or prophylaxis of Crohn's disease or ulcerative colitis is an antibody that interacts with (*i.e.* binds to or recognises) CSF-1 or its receptor and inhibits the activity of CSF-1. Accordingly, there is provided the use of an antibody that inhibits the activity of CSF-1 for the manufacture of a medicament for use in the treatment and/or prophylaxis of Crohn's disease or ulcerative colitis.

In one example the antibodies selectively interact with CSF-1. Selectively interacting with (*e.g*. recognising or binding to) means that the antibodies have a greater affinity for CSF-1 polypeptides than for other polypeptides. Examples of suitable antibodies are those that inhibit the activity of CSF-1 by binding to CSF-1 in such a manner as to prevent it being biologically active by preventing the binding of CSF-1 to its receptor. Accordingly, there is provided by the present invention the use of an anti-CSF-1 antibody for the manufacture of a medicament for use in the treatment and/or prophylaxis of Crohn's disease or ulcerative colitis.

In another example the antibodies selectively interact with the CSF-1 receptor. Selectively interacting with (*e.g*. recognising or binding to) means that the antibodies have a greater affinity for the CSF-1 receptor polypeptide than for other polypeptides. Examples of suitable antibodies are those that inhibit the activity of CSF-1 by preventing CSF-1 mediated signalling from the receptor by preventing CSF-1 from binding to the CSF-1 receptor. Accordingly, there is provided by the present invention the use of an anti-CSF-1R antibody for the manufacture of a medicament for use in the treatment and/or prophylaxis of Crohn's disease or ulcerative colitis.

CSF-1 or CSF-1 receptor polypeptides or cells expressing said polypeptides can be used to produce antibodies which specifically recognise said polypeptides. The CSF-1 and CSF-1 R polypeptides may be 'mature' polypeptides or biologically active fragments or derivatives thereof. Preferably the CSF-1 polypeptide contains amino acids 1-149 believed to be important for biological activity. CSF-1 and CSF-1 R polypeptides may be prepared by processes well known in the art from genetically engineered host cells comprising expression systems or they may be recovered from natural biological sources. In the present application, the term "polypeptides" includes peptides, polypeptides and proteins. These are used interchangeably unless otherwise specified. CSF-1 or CSF-1R polypeptides may in some instances be part of a larger protein such as a fusion protein for example fused to an affinity tag. Antibodies generated against these polypeptides may be obtained by administering the polypeptides to an animal, preferably a non-human animal, using well-known and routine protocols, see for example Handbook of Experimental Immunology, D. M. Weir (ed.), Vol 4, Blackwell Scientific Publishers, Oxford, England, 1986). Many warm-blooded animals, such as rabbits, mice, rats, sheep, cows or pigs may be immunized. However, mice, rabbits, pigs and rats are generally preferred.

Anti-CSF-1 and anti-CSF-1 receptor antibodies for use in the present invention include whole antibodies and functionally active fragments or derivatives thereof and may be, but are not limited to, polyclonal, monoclonal, multi-valent, multi-specific, humanized or chimeric antibodies, single chain antibodies, Fab fragments, Fab' and F(ab')₂ fragments, fragments produced by a Fab expression library, anti-idiotypic (anti-Id) antibodies, and epitope-binding fragments of any of the above.

Antibodies include immunoglobulin molecules and immunologically active portions of immunoglobulin molecules, *i.e.* molecules that contain an antigen binding site that specifically binds an antigen. The immunoglobulin molecules of the invention can be of any class (*e.g.* IgG, IgE, IgM, IgD and IgA) or subclass of immunoglobulin molecule.

Monoclonal antibodies may be prepared by any method known in the art such as the hybridoma technique (Kohler & Milstein, 1975, Nature, 256:495-497), the trioma technique, the human B-cell hybridoma technique (Kozbor et al., 1983, Immunology Today, 4:72) and the EBV-hybridoma technique (Cole et al., Monoclonal Antibodies and Cancer Therapy, pp77-96, Alan R Liss, Inc., 1985).

Antibodies for use in the invention may also be generated using single lymphocyte antibody methods by cloning and expressing immunoglobulin variable region cDNAs generated from single lymphocytes selected for the production of specific antibodies by for example the methods described by Babcook, J. et al., 1996, Proc. Natl. Acad. Sci. USA 93(15):7843-7848, WO92/02551 and WO2004051268.

Humanized antibodies are antibody molecules from non-human species having one or more complementarity determining regions (CDRs) from the non-human species and a framework region from a human immunoglobulin molecule (see, *e.g.* US 5,585,089).

Chimeric antibodies are those antibodies encoded by immunoglobulin genes that have been genetically engineered so that the light and heavy chain genes are composed of immunoglobulin gene segments belonging to different species. These chimeric antibodies are likely to be less antigenic. Bivalent antibodies may be made by methods known in the art (Milstein et al., 1983, Nature 305:537-539; WO 93/08829, Traunecker et al., 1991, EMBO J. 10:3655-3659). Multi-valent antibodies may comprise multiple specificities or may be monospecific (see for example WO 92/22853).

The antibodies for use in the present invention can also be generated using various phage display methods known in the art and include those disclosed by Brinkman et al. (in J. Immunol. Methods, 1995, 182: 41-50), Ames et al. (J. Immunol. Methods, 1995, 184:177-186), Kettleborough et al. (Eur. J. Immunol. 1994, 24:952-958), Persic et al. (Gene, 1997 187 9-18), Burton et al. (Advances in Immunology, 1994, 57:191-280) and WO 90/02809; WO 91/10737; WO 92/01047; WO 92/18619; WO 93/11236; WO 95/15982; WO 95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108. Techniques for the production of single chain antibodies, such as those described in US 4,946,778 can also be adapted to produce single chain antibodies to CSF-1 or CSF-1R polypeptides. Also, transgenic mice, or other organisms, including other mammals, may be used to express humanized antibodies.

Antibody fragments and methods of producing them are well known in the art, see for example Verma et al., 1998, Journal of Immunological Methods, 216, 165-181.

Particular examples of antibody fragments for use in the present invention are Fab' fragments which possess a native or a modified hinge region. A number of modified hinge regions have already been described, for example, in US 5,677,425, WO9915549, and WO9825971.

Further examples of particular antibody fragments for use in the present invention include those described in International patent applications PCT/GB2004/002810, PCT/GB2004/002870 and PCT/GB2004/002871 (all filed on 1st July 2004). In particular the modified antibody Fab fragments described in International patent application PCT/GB2004/002810 are preferred.

If desired an antibody for use in the present invention may be conjugated to one or more effector molecule(s). The term effector molecule as used herein includes, for example, antineoplastic agents, drugs, toxins, biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy. In one example, anti-CSF-1 or anti CSF-1 R antibodies can be conjugated to an effector molecule, such as a cytotoxic agent, a radionuclide or drug moiety to modify a given biological response. For example, the therapeutic agent may be a drug moiety which may be a protein or polypeptide possessing a desired biological activity. Such moieties may include, for example and without limitation, a toxin such as abrin, ricin A, pseudomonas exotoxin, or diphtheria toxin, a protein such as tumour necrosis factor, α-interferon, β-interferon, nerve growth factor, platelet derived growth factor or tissue plasminogen activator, a thrombotic agent or an anti-angiogenic agent, *e.g*. angiostatin or endostatin, or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

In another example the effector molecules may be cytotoxins or cytotoxic agents including any agent that is detrimental to (*e.g.* kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Effector molecules also include, but are not limited to, antimetabolites (*e.g.* methotrexate, 6-mercaptopurine, 6-thioguanine, cytarabine, 5-fluorouracil decarbazine), alkylating agents (*e.g.* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.* daunorubicin (formerly daunomycin) and doxorubicin), antibiotics (*e.g.* dactinomycin (formerly actinomycin), bleomycin, mithramycin, anthramycin (AMC), calicheamicins or duocarmycins), and anti-mitotic agents (*e.g.* vincristine and vinblastine).

Other effector molecules may include radionuclides such as ¹¹¹In and ⁹⁰Y, Lu¹⁷⁷, Bismuth²¹³, Californium²⁵², Iridium¹⁹² and Tungsten¹⁸⁸/Rhenium¹⁸⁸; or drugs such as alkylphosphocholines, topoisomerase I inhibitors, taxoids and suramin. Techniques for conjugating such effector molecules to antibodies are well known in the art (see, Hellstrom et al., Controlled Drug Delivery, 2nd Ed., Robinson et al., eds., 1987, pp. 623-53; Thorpe et al., 1982, Immunol. Rev., 62:119-58 and Dubowchik et al., 1999, Pharmacology and Therapeutics, 83, 67-123). In one example, the antibody or fragment thereof is fused via a covalent bond (*e.g.* a peptide bond), at optionally the N-terminus or the C-terminus, to an amino acid sequence of another protein (or portion thereof; preferably at least a 10, 20 or 50 amino acid portion of the protein). Preferably the antibody, or fragment thereof, is linked to the other protein at the N-terminus of the constant domain of the antibody. Recombinant DNA procedures may be used to create such fusions, for example as described in WO 86/01533 and EP 0392745.

In another example the effector molecule may increase half-life *in vivo,* and/or decrease immunogenicity and/or enhance the delivery of an antibody across an epithelial barrier to the immune system. Examples of suitable effector molecules include polymers and proteins such as albumin and albumin binding proteins. Examples of suitable polymers include any synthetic or naturally occurring substantially water-soluble, substantially non-antigenic polymer including, optionally substituted straight or branched chain polyalkylene, polyalkenylene, or polyoxyalkylene polymers or branched or unbranched polysaccharides, e.g. a homo- or hetero- polysaccharide such as lactose, amylose, dextran or glycogen. Particular optional substituents which may be present on the above-mentioned synthetic polymers include one or more hydroxy, methyl or methoxy groups. Particular examples of synthetic polymers include optionally substituted straight or branched chain poly(ethyleneglycol), poly(propyleneglycol), poly(vinylalcohol) or derivatives thereof, especially optionally substituted poly(ethyleneglycol) such as methoxypoly(ethyleneglycol). Preferably the polymer is a polyalkylene oxide such as polyethylene glycol (PEG).

In one example antibodies for use in the present invention are attached to poly(ethyleneglycol) (PEG) moieties. In one particular example the antibody is an antibody fragment and the PEG molecules may be attached through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods. See for example US 5,219,996. Multiple sites can be used to attach two or more PEG molecules. Preferably PEG molecules are covalently linked through a thiol group of at least one cysteine residue located in the antibody fragment. Where a thiol group is used as the point of attachment appropriately activated effector molecules, for example thiol selective derivatives such as maleimides and cysteine derivatives may be used.

Preferably, the antibody is a modified Fab fragment which is PEGylated, *i.e.* has PEG (poly(ethyleneglycol)) covalently attached thereto, *e.g*. according to the method disclosed in EP 0948544 and International patent applications PCT/GB2004/002810, PCT/GB2004/002870 and PCT/GB2004/002871 (all filed on 1st July 2004) [see also "Poly(ethyleneglycol) Chemistry, Biotechnical and Biomedical Applications", 1992, J. Milton Harris (ed), Plenum Press, New York, "Poly(ethyleneglycol) Chemistry and Biological Applications", 1997, J. Milton Harris and S. Zalipsky (eds), American Chemical Society, Washington DC and "Bioconjugation Protein Coupling Techniques for the Biomedical Sciences", 1998, M. Aslam and A. Dent, Grove Publishers, New York; Chapman, A. 2002, Advanced Drug Delivery Reviews 2002, 54:531-545]. The total amount of PEG attached to the fragment may be varied as desired, but will generally be in an average molecular weight range from 250 to 100,000Da, preferably from 5,000 to 50,000Da, more preferably from 10,000 to 40,000Da and still more preferably from 20,000 to 40,000Da. The size of PEG may in particular be selected on the basis of the intended use of the product, for example ability to localize to certain tissues such as tumors or extend circulating half-life (for review see Chapman, 2002, Advanced Drug Delivery Reviews, 54, 531-545).

Preferably the antibody is a modified Fab' fragment and PEG is attached to a cysteine in the hinge region. In one example, a PEG modified Fab' fragment has a maleimide group covalently linked to a single thiol group in a modified hinge region. A lysine residue may be covalently linked to the maleimide group and to each of the amine groups on the lysine residue may be attached a methoxypoly(ethyleneglycol) polymer having a molecular weight of approximately 20,000 Da. The total molecular weight of the PEG attached to the Fab' fragment may therefore be approximately 40,000 Da.

In another preferred embodiment an antibody fragment for use in the present invention is a PEGylated (i.e. has PEG (poly(ethyleneglycol)) covalently attached thereto) Fab fragment as described in International Application Number PCT/GB2004/002810 (filed on 1st July 2004). Preferably the PEGylated Fab fragment has PEG attached to each interchain cysteine. Preferably each PEG attached to an interchain cysteine has a molecular weight of 20,000Da and the total molecular weight of the PEG attached to the Fab fragment is 40,000Da.

To identify inhibitors of CSF-1 activity a number of different approaches may be taken by those skilled in the art. In one example inhibitors are identified by first identifying agents that interact with CSF-1 or CSF-1R and subsequently testing those agents to identify those that inhibit CSF-1 activity. In one such example the agent is an antibody.

Agents that interact with CSF-1 or CSF1-R may be identified using any suitable method, for example by using a cell-free or cell-based assay system where the CSF-1 or CSF-1R polypeptide is contacted with a candidate agent and the ability of the candidate agent to interact with the polypeptide is determined. Preferably, the ability of a candidate agent to interact with a CSF-1 or CSF-1R polypeptide is compared to a reference range or control. If desired, this assay may be used to screen a plurality (*e.g.* a library) of candidate agents using a plurality of CSF-1 or CSF-1R polypeptide samples. In one example of a cell free assay, a first and second sample comprising native or recombinant CSF-1 or CSF-1R polypeptide are contacted with a candidate agent or a control agent and the ability of the candidate agent to interact with the polypeptide is determined by comparing the difference in interaction between the candidate agent and control agent. Preferably, the polypeptide is first immobilized, by, for example, contacting the polypeptide with an immobilized antibody which specifically recognizes and binds it, or by contacting a purified preparation of polypeptide with a surface designed to bind proteins. The polypeptide may be partially or completely purified (*e.g.* partially or completely free of other polypeptides) or part of a cell lysate. Further, the polypeptide may be a fusion protein comprising the CSF-1 or CSF1-R polypeptide or a biologically active portion thereof and a domain such as glutathionine-S-transferase or the Fc region of IgG1. Alternatively, the polypeptide can be biotinylated using techniques well known to those of skill in the art (*e.g.* biotinylation kit, Pierce Chemicals; Rockford, IL). The ability of the candidate agent to interact with the polypeptide can be determined by methods known to those of skill in the art for example, ELISA, BIAcore™, Flow cytometry or fluorescent microvolume assay technology (FMAT). In another example where a cell-based assay is used, a population of cells expressing CSF-1 or CSF-1 R is contacted with a candidate agent and the ability of the candidate agent to interact with the polypeptide is determined. Preferably, the ability of a candidate agent to interact with CSF-1 or CSF-1 R is compared to a reference range or control. The cell can be of eukaryotic origin (*e.g.* yeast or mammalian) and can express the CSF-1 or CSF-1 R polypeptide endogenously or be genetically engineered to express the polypeptide. In some instances, the CSF-1 or CSF-1R polypeptide or the candidate agent is labelled, for example with a radioactive label (such as ³²P, ³⁵S or ¹²⁵I) or a fluorescent label (such as fluorescein isothiocyanate, rhodamine, phycoerythrin, phycocyanin, allophycocyanin, o-phthaldehyde or fluorescamine) to enable detection of an interaction between a polypeptide and a candidate agent. Alternative methods such as ELISA, flow cytometry and FMAT may also be used.

Agents which inhibit CSF-1 activity may be identified by any suitable method, for example by:
(i) comparing the activity of CSF-1 in the presence of a candidate agent with the activity of said polypeptide in the absence of the candidate agent or in the presence of a control agent; and
(ii) determining whether the candidate agent inhibits activity of CSF-1

Such assays can be used to screen candidate agents, in clinical monitoring or in drug development.

As described above, agents may be pre-screened where appropriate (e.g. an antibody) to identify agents that interact with CSF-1 or CSF-1R prior to screening those agents which bind for their ability to inhibit CSF-1 activity.

In one example a cell-based assay system is used to identify agents capable of inhibiting the activity of CSF-1. In one particular example the assay used to identify inhibitors of CSF-1 activity is the standard *in vitro* colony stimulating assay of Metcalf, 1970, J. Cell.Physiol.76-89 in which CSF-1 is capable of stimulating the formation of macrophage colonies. Potential inhibitors are added to the assay and proliferation of macrophages is measured by any suitable method such as ³H thymidine incorporation or formazan dye conversion. Inhibition is therefore measured as a reduction in proliferation compared to controls.

In another example inhibitors of CSF-1 may down-regulate the expression of the CSF-1 or CSF-1R polypeptide, for example antisense inhibitors. Such inhibitors may be identified by any method known in the art. In one example such inhibitors are identified in a cell-based assay system. Accordingly, a population of cells expressing a CSF-1 or CSF-R polypeptide or nucleic acid are contacted with a candidate agent and the ability of the candidate agent to alter expression of the CSF-1 or CSF-1R polypeptide or nucleic acid is determined by comparison to a reference range or control. In one example, populations of cells expressing a CSF-1 or CSF1-R polypeptide are contacted with a candidate agent or a control agent and the ability of the candidate agent to alter the expression of the CSF-1 or CSF-1R polypeptides or nucleic acids is determined by comparing the difference in the level of expression of the CSF-1 or CSF1-R polypeptides or nucleic acids between the treated and control populations of cells. If desired, this assay may be used to screen a plurality (e.g. a library) of candidate agents. The cell, can be of eukaryotic origin (*e.g.* yeast or mammalian) and can express a CSF-1 or CSF-1R polypeptide endogenously or be genetically engineered to express a CSF-1 or CSF-1R polypeptide. The ability of the candidate agents to alter the expression of a said polypeptides or nucleic acids can be determined by methods known to those of skill in the art, for example and without limitation, by flow cytometry, radiolabelling, a scintillation assay, immunoprecipitation, Western blot analysis, Northern blot analysis or RT-PCR.

Agents that inhibit the activity of CSF-1 may be identified or further tested, to determine therapeutically effective amounts in one or more animal models. Examples of suitable animals include, but are not limited to, mice, rats, rabbits, monkeys, guinea pigs, dogs and cats. Preferably, the animal used represents a model of IBD.

In one example where the agent inhibits the expression of CSF-1 or CSF-1R, a first and second group of mammals are administered with a candidate agent or a control agent and the ability of the candidate agent to inhibit the expression of CSF-1 or CSF-1R polypeptide or nucleic acid is determined by comparing the difference in the level of expression between the first and second group of mammals. Where desired, the expression levels of the CSF-1 or CSF-1R polypeptides or nucleic acid in the first and second groups of mammals can be compared to the level of CSF-1 or CSF-1R polypeptide or nucleic acid in a control group of mammals. The candidate agent or a control agent can be administered by means known in the art (*e.g.* orally, rectally or parenterally such as intraperitoneally or intravenously). Changes in the expression of a polypeptide or nucleic acid can be assessed by the methods outlined above.

In another example, the inhibition of CSF-1 activity can be determined by monitoring an amelioration or improvement in disease symptoms, a delayed onset or slow progression of the disease, for example but without limitation, a reduction in weight loss or diarrhoea. Techniques known to physicians familiar with IBD can be used to determine whether a candidate agent has altered one or more symptoms associated with the disease.

A number of different models of IBD are known in the art. These include DSS-induced colitis in mice or rats, TNBS-induced colitis in mice or rats, CD45RBhi cell transfer into SCID or RAG1-/- mice, IL-10-/- mice, IL2-/- mice, HLA-B27 transgenic rats, indomethacin-induced enteritis in rats and cotton top tamarins (See Elson et al., Gastroenterology, 1995; 109, 1344-1367; Mizoguchi et al., Inflammatory Bowel Disease, 2003; 9 (4), 246-259). Generally the different models tend to show signs of colitis such as weight loss and diarrhoea with or without the presence of blood. The intestines will usually show an inflammatory infiltrate and the extent of this and the exact site of disease can vary between models. The symptoms that are measured during treatment will therefore vary between models but will typically include measuring weight loss or the presence of diarrhoea and may also include looking for histological improvements, such as reduced inflammatory infiltrate or reduced damage to the crypts.

As discussed herein, inhibitors of CSF-1 activity can be used in the treatment and/or prophylaxis of Crohn's disease or ulcerative colitis. For such use the agents will generally be administered in the form of a pharmaceutical composition.

Also provided is a pharmaceutical composition comprising an inhibitor of CSF-1 activity and a pharmaceutically acceptable diluent, excipient and /or carrier.

The term 'treatment' includes either therapeutic or prophylactic therapy. When a reference is made herein to a method of treating or preventing a disease or condition using a particular inhibitor or combination of inhibitors, it is to be understood that such a reference is intended to include the use of that inhibitor or combination of inhibitors for the manufacture of a medicament for the treatment and/or prophylaxis of Crohn's disease or ulcerative colitis.

The composition will usually be supplied as part of a sterile, pharmaceutical composition that will normally include a pharmaceutically acceptable carrier. This composition may be in any suitable form (depending upon the desired method of administering it to a patient).

The inhibitors of use in the invention are preferably administered to a subject orally or intrarectally but may also be administered by a variety of other routes such as transdermally, subcutaneously, intranasally, intravenously and intramuscularly. The most suitable route for administration in any given case will depend on the particular inhibitor, the subject, and the nature and severity of the disease and the physical condition of the subject.

The inhibitors of use in the invention may be administered in combination, *e.g.* simultaneously, sequentially or separately, with one or more other therapeutically active compounds, which may be anti-IBD therapies or anti-cancer therapies.

Pharmaceutical compositions may be conveniently presented in unit dose forms containing a predetermined amount of an active agent of the invention per dose. Such a unit may contain 750mg/kg to 0.1mg/kg depending on the condition being treated, the route of administration and the age, weight and condition of the subject.

Pharmaceutically acceptable carriers for use in the invention may take a wide variety of forms depending, *e.g.* on the route of administration.

Compositions for oral administration may be liquid or solid. Oral liquid preparations may be in the form of aqueous or oily suspensions, solutions, emulsions, syrups or elixirs, or may be presented as a dry product for reconstitution with water or other suitable vehicle before use. Oral liquid preparations may contain suspending agents as known in the art.

In the case of oral solid preparations such as powders, capsules and tablets, carriers such as starches, sugars, microcrystalline cellulose, granulating agents, lubricants, binders, disintegrating agents may be included. Because of their ease of administration, tablets and capsules represent the most advantageous oral dosage unit form in which case solid pharmaceutical carriers are generally employed. In addition to the common dosage forms set out above, active agents of the invention may also be administered by controlled release means and/or delivery devices. Tablets and capsules may comprise conventional carriers or excipients such as binding agents for example, syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate, talc, polyethylene glycol or silica; disintegrants, for example potato starch; or acceptable wetting agents such as sodium lauryl sulphate. The tablets may be coated by standard aqueous or non-aqueous techniques according to methods well known in normal pharmaceutical practice.

Pharmaceutical compositions of the present invention suitable for oral administration may be presented as discrete units such as capsules, cachets or tablets, each containing a predetermined amount of the active agent, as a powder or granules, or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active agent with the carrier, which constitutes one or more necessary ingredients. In general, the compositions are prepared by uniformly and intimately admixing the active agent with liquid carriers or finely divided solid carriers or both, and then, if necessary, shaping the product into the desired presentation. For example, a tablet may be prepared by compression or moulding, optionally with one or more accessory ingredients.

Pharmaceutical compositions suitable for parenteral administration may be prepared as solutions or suspensions of the active agents of the invention in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include aqueous or non-aqueous sterile injection solutions which may contain anti-oxidants, buffers, bacteriostats and solutes which render the composition isotonic with the blood of the intended recipient, and aqueous and non-aqueous sterile suspensions which may include suspending agents and thickening agents. Extemporaneous injection solutions, dispersions and suspensions may be prepared from sterile powders, granules and tablets.

Pharmaceutical compositions can be administered with medical devices known in the art. In a preferred embodiment, a pharmaceutical composition of the invention can be administered with a needleless hypodermic injection device, such as the devices disclosed in US 5,399,163; 5,383,851; 5,312,335; 5,064,413; 4,941,880; 4,790,824; or 4,596,556. Examples of well-known implants and modules useful in the present invention include: US 4,487,603, which discloses an implantable micro-infusion pump for dispensing medication at a controlled rate; US 4,486,194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,196, which discloses an osmotic drug delivery system. Many other such implants, delivery systems, and modules are known to those skilled in the art.

Pharmaceutical compositions adapted for topical administration may be formulated as ointments, creams, suspensions, lotions, powders, solutions, pastes, gels, impregnated dressings, sprays, aerosols or oils, transdermal devices, dusting powders. These compositions may be prepared via conventional methods containing the active agent. Thus, they may also comprise compatible conventional carriers and additives, such as preservatives, solvents to assist drug penetration, emollients in creams or ointments and ethanol or oleyl alcohol for lotions. Such carriers may be present as from 1% up to 98% of the composition. More usually they will form up to 80% of the composition. As an illustration only, a cream or ointment is prepared by mixing sufficient quantities of hydrophilic material and water, containing from 5-10% by weight of the compound, in sufficient quantities to produce a cream or ointment having the desired consistency.

Pharmaceutical compositions adapted for transdermal administration may be presented as discrete patches intended to remain in intimate contact with the epidermis of the recipient for a prolonged period of time. The active agent may be delivered from the patch by iontophoresis.

For applications to external tissues, for example the mouth and skin, the compositions are preferably applied as a topical ointment or cream. When formulated in an ointment, the active agent may be employed with either a paraffinic or a water-miscible ointment base. Alternatively, the active agent may be formulated in a cream with an oil-in-water cream base or a water-in-oil base.

Pharmaceutical compositions adapted for topical administration in the mouth include lozenges, pastilles and mouth washes.

Pharmaceutical compositions adapted for topical administration to the eye include eye drops wherein the active agent is dissolved or suspended in a suitable carrier, especially an aqueous solvent. They also include topical ointments or creams as above.

Pharmaceutical compositions suitable for rectal administration wherein the carrier is a solid are most preferably presented as unit dose suppositories. Suitable carriers include cocoa butter or other glyceride or materials commonly used in the art, and the suppositories may be conveniently formed by admixture of the combination with the softened or melted carrier(s) followed by chilling and shaping moulds. They may also be administered as enemas.

The dosage to be administered of an inhibitor of CSF-1 activity will vary according to the particular inhibitor, the type of IBD, the subject, and the nature and severity of the disease and the physical condition of the subject, and the selected route of administration; the appropriate dosage can be readily determined by a person skilled in the art. For the treatment and/or prophylaxis of Crohn's disease or ulcerative colitis in humans and animals pharmaceutical compositions comprising antibodies can be administered to patients (*e.g.,* human subjects) at therapeutically or prophylactically effective dosages (*e.g.* dosages which result in inhibition of Crohn's disease or ulcerative colitisand/or relief of Crohn's disease or ulcerative colitis symptoms) using any suitable route of administration, such as injection and other routes of administration known in the art for clinical products, such as antibody-based clinical products.

The compositions may contain from 0.1% by weight, preferably from 10-60%, or more, by weight, of the inhibitor of the invention, depending on the method of administration.

It will be recognized by one of skill in the art that the optimal quantity and spacing of individual dosages of an inhibitor of the invention will be determined by the nature and extent of the condition being treated, the form, route and site of administration, and the age and condition of the particular subject being treated, and that a physician will ultimately determine appropriate dosages to be used. This dosage may be repeated as often as appropriate. If side effects develop the amount and/or frequency of the dosage can be altered or reduced, in accordance with normal clinical practice.

In another example, where the inhibitor is a nucleic acid this may be administered via gene therapy (see for example Hoshida, T. et al., 2002, Pancreas, 25:111-121; Ikuno, Y. 2002, Invest. Ophthalmol. Vis. Sci. 2002 43:2406-2411; Bollard, C., 2002, Blood 99:3179-3187; Lee E., 2001, Mol. Med. 7:773-782). Gene therapy refers to administration to a subject of an expressed or expressible nucleic acid. In one example this is either the CSF-1 or the CSF-1R nucleic acid or portions thereof. Any of the methods for gene therapy available in the art can be used according to the present invention.

Delivery of the therapeutic nucleic acid into a patient can be direct *in vivo* gene therapy (*i.e*. the patient is directly exposed to the nucleic acid or nucleic acid-containing vector) or indirect *ex vivo* gene therapy (*i.e.* cells are first transformed with the nucleic acid *in vitro* and then transplanted into the patient).

For example for *in vivo* gene therapy, an expression vector containing the CSF-1 or CSF-1R nucleic acid may be administered in such a manner that it becomes intracellular, *i.e.* by infection using a defective or attenuated retroviral or other viral vectors as described, for example, in US 4,980,286 or by Robbins et al., 1998, Pharmacol. Ther. 80:35-47.

The various retroviral vectors that are known in the art are such as those described in Miller et al. (1993, Meth. Enzymol. 217:581-599) which have been modified to delete those retroviral sequences which are not required for packaging of the viral genome and subsequent integration into host cell DNA. Also adenoviral vectors can be used which are advantageous due to their ability to infect non-dividing cells and such high-capacity adenoviral vectors are described in Kochanek (1999, Human Gene Therapy, 10:2451-2459). Chimeric viral vectors that can be used are those described by Reynolds et al. (1999, Molecular Medicine Today, 1:25 -31). Hybrid vectors can also be used and are described by Jacoby et al. (1997, Gene Therapy, 4:1282-1283).

Direct injection of naked DNA or through the use of microparticle bombardment (*e.g*. Gene Gun®; Biolistic, Dupont) or by coating it with lipids can also be used in gene therapy. Cell-surface receptors/transfecting compounds or through encapsulation in liposomes, microparticles or microcapsules or by administering the nucleic acid in linkage to a peptide which is known to enter the nucleus or by administering it in linkage to a ligand predisposed to receptor-mediated endocytosis (See Wu & Wu, 1987, J. Biol. Chem., 262:4429-4432) can be used to target cell types which specifically express the receptors of interest.

In *ex vivo* gene therapy, a gene is transferred into cells *in vitro* using tissue culture and the cells are delivered to the patient by various methods such as injecting subcutaneously, application of the cells into a skin graft and the intravenous injection of recombinant blood cells such as haematopoietic stem or progenitor cells.

Cells into which a CSF-1 or CSF-1R nucleic acid can be introduced for the purposes of gene therapy include epithelial cells, endothelial cells, keratinocytes, fibroblasts, muscle cells, hepatocytes and blood cells. The blood cells that can be used include, T-lymphocytes, B-lymphocytes, monocytes, macrophages, neutrophils, eosinophils, megakaryotcytes, granulocytes, haematopoietic cells or progenitor cells.

In a one aspect, the pharmaceutical composition of the present invention comprises a CSF-1 or CSF-1R nucleic acid, said nucleic acid being part of an expression vector that expresses a CSF1 or CSF-1R polypeptide or chimeric protein thereof in a suitable host. In particular, such a nucleic acid has a promoter operably linked to the polypeptide coding region, said promoter being inducible or constitutive (and, optionally, tissue-specific).

The invention will now be described with reference to the following examples.
Figure 1. Effect of anti-CSF-1 antibody on body weight of mice after addition of DSS (1%) in drinking water. Statistical analysis by ANOVA with Bonferroni's post test:- **P<0.01, ***P<0.001 control antibody (101.4) vs. normal animals. #P<0.05, ##P<0.01, ###P<0.001 101.4 vs. anti CSF-1.
Figure 2. Effect of anti-CSF-1 antibody on colon length of mice after addition of DSS (1%) in drinking water for 8 days. Statistical analysis by ANOVA with Bonferroni's post test.
Figure 3a. Effect of anti-CSF-1 antibody on colonic disease severity score of mice after addition of DSS (1%) in drinking water. Disease score: 1 = soft faeces/diarrhoea; 2 = signs of blood in gut/faeces; 4 = profuse bleeding from anus. **P<0.01 analysed by Mann Whitney.
Figure 3b. Effect of anti-CSF-1 antibody on clinical disease severity score of mice after addition of DSS (1%) in drinking water. Disease scored as gut disease score plus weight loss score: gut disease score: 1 = soft faeces/diarrhoea; 2 = signs of blood in gut/faeces; 4 = profuse bleeding from anus. Weight loss score: 1 = 5-10%; 2 = 10-15%; 4=15-25% weight loss. **P<0.01; ***P<0.01 analysed by Mann Whitney.
Figure 4a. Effect of anti-CSF-1 antibody on GR1 high+ CD45+cells/mm in lamina propria population from colons of mice on day 8 after DSS(1%). Statistical analysis by ANOVA with Bonferroni's post test.
Figure 4b. Effect of anti-CSF-1 antibody on CD3+ CD45+ cells/mm in lamina propria population from colons of mice on day 8, after DSS (1%). Statistical analysis by ANOVA with Bonferroni's post test.
Figure 5. Effect of anti-CSF-1 antibody on CD3+ cell count from colons of mice on day 8 after DSS(1%). Statistical analysis by ANOVA with Bonferroni's post test.
Figure 6. Effect of anti-CSF-1 antibody on F4/80+ cell count from colons of mice on day 8, after DSS(1%). Statistical analysis by ANOVA with Bonferroni's post test.

### Examples

### Example 1. Isolation of an anti-CSF-1 antibody

Rabbits were immunised with mouse CSF-1 and using a haemolytic plaque assay with biotinylated sheep red blood cells coated with murine CSF-1 via streptavidin, 15 antibody genes were isolated using the methods described by Babcook et al., 1996, Proc.Natl.Acad.Sci, 93, 7843-7848 and in WO92/02551. The antibody genes were expressed in CHO cells and the recombinant antibodies screened for their ability to neutralise murine CSF-1 in a M-NFS-60 cell proliferation assay (Metcalf, J.Cell.Physiol (1970) 76-89). M-NFS-60 is a mouse macrophage cell line that is dependent on CSF-1 for growth. One antibody which neutralised CSF-1 activity was selected, mCSF1033 and a chimeric IgG (Ab33) produced using the rabbit variable regions from antibody mCSF1033 and mouse constant regions. The free cysteine residue in the rabbit variable region was alkylated using iodoacetamide (Pierce). This chimeric antibody was then used for testing in *in vivo* mouse models of IBD.

### Example 2. Effect of anti-CSF-1 antibody on the symptoms of DSS induced acute colitis

The dextran sulfate sodium (DSS) model used was essentially as described in Cooper et al., 1993, Laboratory Investigation, 69, 238-249. Two groups of 10 male Balb/c mice were weighed and injected subcutaneously with either Ab33 (see Example 1) or a control antibody 101.4 (mouse anti-human TNFalpha antibody) at 10mg/kg. Normal drinking water was then replaced with 1% DSS in tap water 24 hours after the first antibody injection. Subsequent injections of antibodies at the same concentration were given once a week. A third group of 10 male Balb/c mice received no treatment and continued to receive normal drinking water.

Animals were weighed every day and signs of disease (loose stools, bleeding) noted. The volume of 1% DSS or water consumed was also measured by weight. At end of the experiment colons were removed and length measured.

A 1 cm section was collected from the distal end for assessment of neutrophil and T cell infiltration by FACS analysis after digestion with 100U/ml collagenase to release lamina propria leukocytes from the tissue. Neutrophils were defined as those cells expressing CD45 and high levels of GR1 and T cells as those expressing CD45 and CD3 these cells were identified by staining with anti-CD45 CyChrome and anti-GR1 Phycoerythrin antibodies or anti-CD45 CyChrome and anti-CD3 FITC antibodies respectively.

The next 2 cm section was collected for histological analysis and embedded in paraffin. For the immunohistochemical analysis 4µm sections were cut from the embedded paraffin samples, mounted on Superfrost Plus Gold slides, de-waxed in Histoclear and rehydrated. Before the immunohistochemical procedure (a streptavidin-biotin-horseradish peroxidase method) heat mediated antigen retrieval with 0.01 mol/L citrate buffer pH 6 (DakoCytomation) was applied for 25 minutes at 99°C. Endogenous peroxidase activity was blocked by incubation in 0.6% vol/vol hydrogen peroxide in absolute methanol for 30 minutes. To block any endogenous avidin-binding activity the sections were treated with Avidin D blocking solution for 15 minutes, rinsed briefly with buffer then incubated for 15 minutes with the biotin blocking solution (Vector Laboratories). The tissue sections were blocked with 20% normal donkey serum (Jackson ImmunoResearch Laboratories, Inc.) in TBS for 20 minutes at room temperature in order to reduce background signals caused by IgG Fc interactions by the biotinylated second step donkey reagent. The primary antibody was a polyclonal goat anti-mouse CD3 (Autogen Bioclear) diluted 1:800 in 0.05M Tris buffered saline pH 7.6 (TBS). The sections were incubated for 60 minutes at room temperature. Thereafter the slides were incubated for 30 minutes at room temperature with biotinylated donkey anti-goat antibody (Jackson ImmunoResearch Laboratories, Inc.) diluted 1:500 in TBS. The slides were subsequently incubated with streptavidin-biotin-horseradish peroxidase complex (DakoCytomation) for 30 minutes at room temperature. The reaction was developed with 3, 3'-diaminobenzidine tetrahydrochloride. Between steps the slides were rinsed for 5 minutes in TBS twice. All sections were lightly counterstained for 20 seconds with haematoxylin, dehydrated and mounted. Control slides were incubated with TBS instead of primary antibody. The CD3 values were determined by one blinded observer by means of an eyepiece graticule and assessed from areas along the colonic lamina propria. The number of CD3 positive cells within each selected area was counted at a magnification of x400 (10/animal). Grid area at x400 corresponds to 0.059mm² of tissue. The immunohistochemical method for the analysis of F4/80 positive staining cells was performed in the same way as for the CD3 analysis, in this case the tissue sections were blocked with 20% normal rabbit serum (DakoCytomation) in TBS for 20 minutes at room temperature in order to reduce background signals caused by IgG Fc interactions by the biotinylated second step rabbit reagent. The primary antibody was a monoclonal rat anti-mouse F4/80 (Serotec) diluted 1:100 in 0.05M Tris buffered saline pH 7.6 (TBS). The sections were incubated for 60 minutes at room temperature. Thereafter the slides were incubated for 30 minutes at room temperature with biotinylated rabbit anti-rat antibody (DakoCytomation) diluted 1:200 in TBS. The method was then the same as for the CD3 positive cell analysis

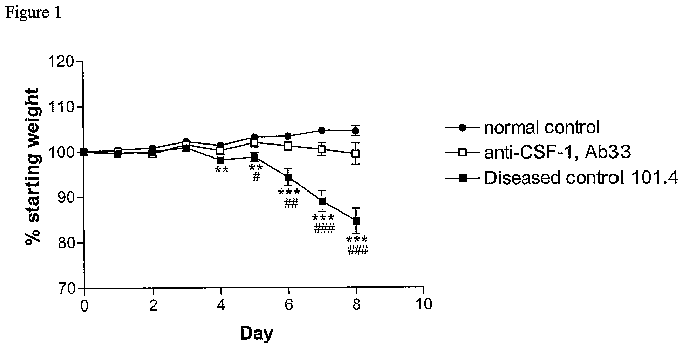

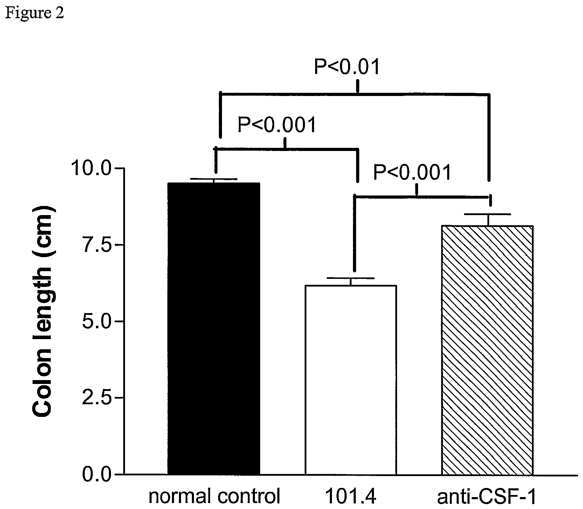

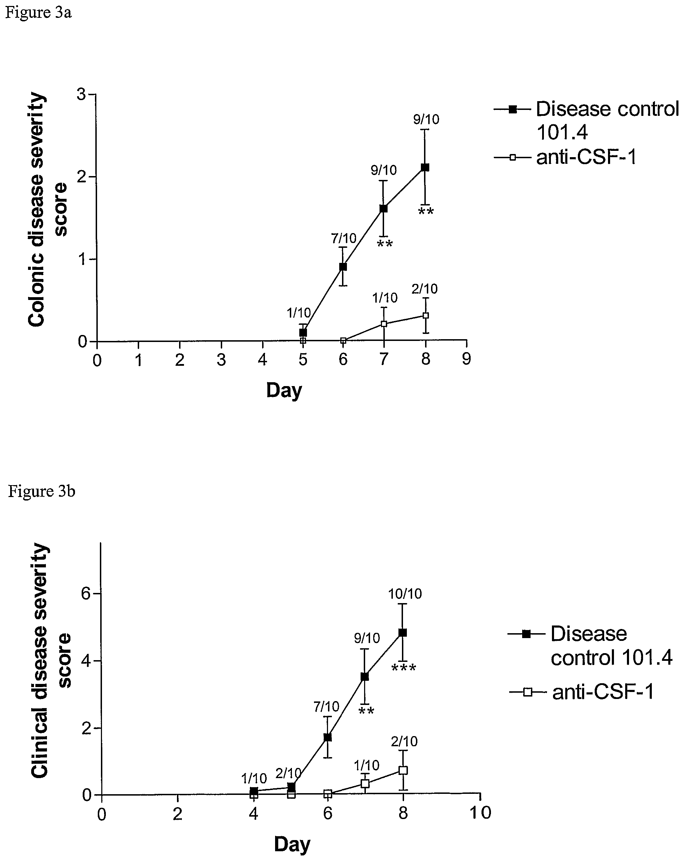

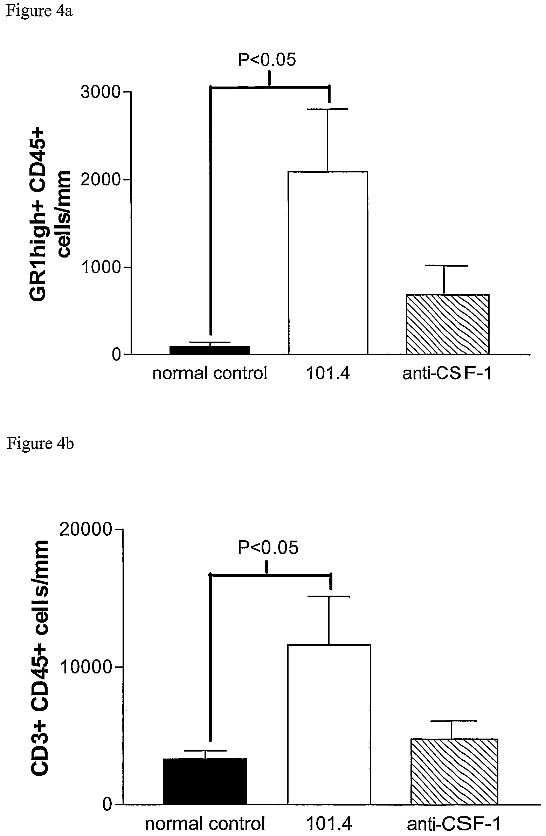

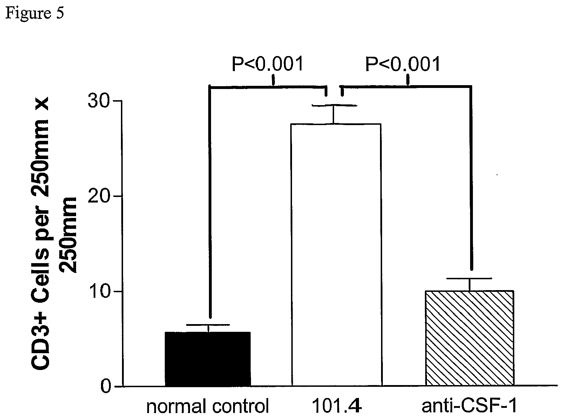

Figure 1 shows following statistical analysis by ANOVA with Bonferroni's post test that there was no significant difference between anti-CSF-1 treated mice and normal control mice indicating that the anti-CSF-1 antibody had prevented the weight loss that normally occurs in DSS-induced colitis (see control antibody 101.4). Figure 2 shows that anti-CSF-1 also had a significant effect on colon length. The length of the colon is significantly reduced in mice with DSS-induced colitis and this is thought to reflect the extent of colonic inflammation. A protection from colon shortening was noted in mice treated with anti-CSF-1 antibody which had a significantly longer colon length than those treated with the negative control antibody 101.4. Figures 3a and 3b show that anti-CSF-1 antibody also reduced the colonic disease severity score (signs of disease such as diarrhoea and rectal bleeding) and overall clinical disease score (a combination of colonic disease and weight loss). FACS analysis of colonic tissue is shown in Figures 4a and 4b. The number of neutrophils (GR1+ CD45+ cells) and T cells (CD3+ CD45+ cells) infiltrating the colon were found to be significantly increased in mice with DSS-induced colitis and treated with the 101.4 negative control antibody, whereas those mice treated with anti-CSF-1 did not have significantly increased numbers of neutrophils or T cells compared with normal control mice. The ability of the anti-CSF-1 antibody to prevent the increased numbers of infiltrating CD3+ T cells into the colonic lamina propria in DSS-colitis was confirmed by immunohistochemistry, as shown in Figure 5.

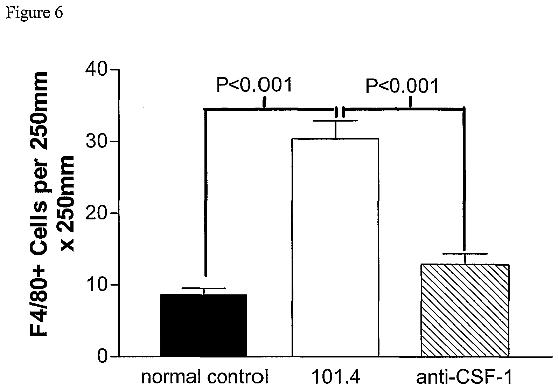

Figure 6 shows that the anti-CSF-1 antibody also inhibited the infiltration of macrophages into the colonic lamina propria as noted by the significant decrease in F4/80 positive staining cells compared with DSS colitis mice treated with 101.4 control antibody.

## Claims

1. The use of an inhibitor of CSF-1 activity for the manufacture of a medicament for the treatment and/or prophylaxis of Crohn's disease or ulcerative colitis wherein the inhibitor is an antibody or functionally active fragment thereof which binds to CSF-1R.

2. The use according to claim 1, wherein the antibody or fragment thereof is monoclonal, polyclonal, chimeric, humanised or bispecific.

3. The use according to claim 1 or claim 2 where the antibody fragment is a Fab, Fab', F(ab')₂, scFv or an epitope-binding fragment thereof.

4. The use according to any one of claims 1-3 wherein the antibody or fragment thereof is conjugated to one or more effector molecule(s).

## Patentansprüche

1. Verwendung eines Inhibitors von CSF-1-Aktivität zur Herstellung eines Arzneimittels für die Behandlung und/oder Prophylaxe von Morbus Crohn oder Colitis ulcerosa, wobei es sich bei dem Inhibitor um einen Antikörper bzw. ein funktionsfähiges Fragment davon, der bzw. das an CSF-1R bindet, handelt.

2. Verwendung nach Anspruch 1, wobei der Antikörper bzw. das Fragment davon monoklonal, polyklonal, chimär, humanisiert oder bispezifisch ist.

3. Verwendung nach Anspruch 1 oder Anspruch 2, wobei es sich bei dem Antikörperfragment um ein Fab, Fab', F(ab')₂, scFv oder ein epitopbindendes Fragment davon handelt.

4. Verwendung nach einem der Ansprüche 1-3, wobei der Antikörper bzw. das Fragment davon an ein oder mehrere Effektormolekül(e) konjugiert ist.

## Revendications

1. Utilisation d'un inhibiteur d'activité CSF-1 pour la fabrication d'un médicament pour le traitement et/ou la prophylaxie de la maladie de Crohn ou de la recto-colite hémorragique, dans laquelle l'inhibiteur est un anticorps ou un fragment fonctionnellement actif de celui-ci qui se lie à CSF-1R.

2. Utilisation selon la revendication 1, dans laquelle l'anticorps ou un fragment de celui-ci est monoclonal, polyclonal, chimérique, humanisé ou bispécifique.

3. Utilisation selon la revendication 1 ou la revendication 2, dans laquelle le fragment d'anticorps est un Fab, Fab', F(ab')₂, scFv ou un fragment de liaison d'épitope de ceux-ci.

4. Utilisation selon l'une quelconque des revendications 1 à 3, dans laquelle l'anticorps ou un fragment de celui-ci est conjugué à une ou plusieurs molécule(s) effectrice(s).
